# EUROPÄISCHE PATENTSCHRIFT

(11) **EP 1 677 691 B1**
(45) Veröffentlichungstag und Bekanntmachung des Hinweises auf die Patenterteilung: **29.09.2010**
(21) Anmeldenummer: 03753210.8
(22) Anmeldetag: 30.10.2003
(51) Int. Cl.: A61B 17/70

(54) **Interspinales Implantat**
Intervertebral implant
Implant intervertébral

(43) Veröffentlichungstag der Anmeldung: 12.07.2006
(73) Patentinhaber: Synthes GmbH, 4436 Oberdorf (CH)
(72) Erfinder: HARTMANN, Stephan, CH-4500 Solothurn (CH); STUDER, Armin, CH-4513 Langendorf (CH)
(74) Vertreter: Lusuardi, Werther
(86) Internationale Anmeldenummer: PCT/CH2003/000706
(87) Internationale Veröffentlichungsnummer: WO 2005/041792

(56) Entgegenhaltungen:
- GB-A- 780 652
- US-B1- 6 312 431
- US-B1- 6 364 883

## Beschreibung

Die Erfindung bezieht sich auf ein interspinales Implantat, gemäss dem Oberbegriff des Patentanspruchs 1.
Solche Implantate können als Abstandhalter für zwei benachbarte Wirbelkörper bei einer defekten Bandscheibe dienen. Der durch die defekte Bandscheibe verringerte Abstand zwischen den Wirbelkörpern lässt sich durch ein solches Implantat wieder vergrössern, so dass auch die Fazetten-Gelenke wieder entlastet werden.

Eine gattungsgemässe interspinale Prothese ist aus der WO 03/015645 MATHIEU bekannt. Diese bekannte interspinale Prothese umfasst ein in den Interspinalraum einzuführendes Mittelstück, aus welchem kranial und kaudal je ein Paar longitudinale Fortsätze rechts und links vom Mittelstück hervorragen, so dass das Mittelstück mittels der Fortsätze zwischen den Dornfortsätzen (processus spinosus) zweier benachbarter Wirbelkörper festgehalten werden kann. Diese bekannte Prothese umfasst jedoch ein Mittelteil, welches nicht expandierbar ist, so dass durch die Prothese selbst keine distrahierte Position der benachbarten Wirbelkörper herstellbar ist.

Aus der WO 98/46173 SCHÄR ist eine mittels eines Ratschenmechanismus teleskopierbare Wirbelprothese bekannt, welche jedoch in den Zwischenwirbelraum eingeführt werden muss, wodurch ein Ausräumen des Zwischenwirbelraumes erforderlich ist. Aus der US 6,364,883 SANTILLI ein interspinales Implantat bekannt, dessen in den Interspinalraum einführbare Bolzen - als solche - nicht distrahierbar sind. Die Vorrichtung von SANTILLI erlaubt keine Distraktion zwischen zwei unmittelbar benachbarten Wirbelkörpern. Auch bei einem aus der GB 780,652 EVANS bekannten interspinalen Implantat sind die einzelnen Bolzen - als solche - nicht distrahierbar.

Hier will die Erfindung Abhilfe schaffen. Der Erfindung liegt die Aufgabe zugrunde, ein interspinales Implantat zu schaffen, welches in den Interspinalraum einführbare Elemente umfasst und zugleich eine Distraktion der angrenzenden Wirbelkörper gestattet und aufrecht erhalten kann.

Die Erfindung löst die gestellte Aufgabe mit einem interspinalen Implantat, welches die Merkmale des Anspruchs 1 aufweist.

Die durch die Erfindung erreichten Vorteile sind im wesentlichen darin zu sehen, dass dank des erfindungsgemässen Implantates:
- kein Ausräumen des Zwischenwirbelraumes erforderlich ist;
- eine Distraktion und Fixierung der angrenzenden Wirbelkörper in einer gewünschten distrahierten Position möglich ist;
- keine Entfernung von Ligamenten, insbesondere dem supraspinalen Ligament erforderlich ist;
- die Distraktion direkt mit dem Implantat erfolgt;
- eine stufenlose Distraktion möglich ist; und
- nur ein Implantat erforderlich ist.

In einer besonderen Ausführungsform sind die zwei relativ zueinander verschiebbaren Dorne mit den hinteren Enden am ersten Teil und mit den vorderen Enden am zweiten Teil in einer gewünschten Position fixierbar. Die Dorne sind vorzugsweise derart ausgestaltet, dass sie für die Implantation eng aneinander zur Anlage bringbar sind. Vorzugsweise sind die Dorne mit einer zur Längsachse orthogonalen, halbkreisförmigen Querschnittsfläche ausgebildet, wobei die ebenen Flächen gegeneinander gerichtet sind. Damit ist der Vorteil erreichbar, dass beim Einbringen der Dorne, insbesondere beim Durchführen der Dorne durch die Ligamente diese und das umliegende Gewebe nur geringfügig beeinträchtigt werden.

In einer anderen Ausführungsform sind die zwei Dorne mit ihren Enden formschlüssig an den zwei Teilen fixierbar, so dass eine unbeabsichtigte Verschiebung der Dorne nach ihrer Distraktion und Fixation verhindert wird.

In wiederum einer anderen Ausführungsform umfasst jedes der zwei Teile ein von seiner Appositionsfläche zu seiner aussenstehenden Oberfläche durchgehendes Langloch, dessen lange Achsen parallel zu den Längsachsen der zwei Teile sind. Die Enden der Dorne sind in den Langlöchern durch die langen Seitenwände der Langlöcher gegen unbeabsichtigte Bewegungen senkrecht zu den langen Achsen der Langlöcher gesichert.

In einer weiteren Ausführungsform ist nur ein Dorn parallel zu den Längsachsen der zwei Teile in den Langlöchern verschiebbar, während der andere Dorn auch vor der Implantation fest mit einem der zwei Teile verbunden ist.

Nachfolgend sind einige geeignete Abmessung für die verschiedenen Bestandteile des interspinalen Implantates angegeben:
- vorzugsweise weist jedes der Teile parallel zu seiner Längsachse eine Höhe H zwischen 10 mm und 30 mm auf;
- vorzugsweise weist jeder der Dorne eine maximale diametrale Abmessung zwischen 2 mm und 5 mm auf;
- die Verschiebbarkeit der zwei Dorne parallel zu den Längsachsen der zwei Teile und relativ zueinander beträgt vorzugsweise zwischen 7 mm und 15 mm.

In wiederum einer weiteren Ausführungsform weist jedes Langloch an seinen zu den Längsachsen der zwei Teile parallelen Seitenflächen eine dreidimensionale, makroskopischen Struktur auf, welche vorzugsweise aus einer Verzahnung besteht. Vorzugsweise sind die zwei Teile aus Titan hergestellt und die zwei Dorne aus einem weicheren Material, so dass das weichere Material der zwei Dorne beispielsweise durch axiales Stauchen der Dorne in die makroskopische Strukturierung an den Seitenflächen der Langlöcher gepresst werden kann. Die Verformung der Dorne ist plastisch, so dass eine formschlüssige Verbindung der Dorne in den Langlöchern der zwei Teile erreichbar ist.

In einer anderen Ausführungsform umfasst mindestens einer der zwei Dorne gegen den Dornfortsatz eines angrenzenden Wirbelkörpers gerichtete, elastische Mittel, so dass der Dorn im Kontaktbereich mit dem angrenzenden Dornfortsatz quer zu seiner Längsachse elastisch deformierbar ist. Damit ist der Vorteil erreichbar, dass das interspinale Implantat nach seiner Implantation zwischen den Dornfortsätzen zweier benachbarter Wirbelkörper parallel zur Wirbelsäulenlängsachse innerhalb eines gewünschten Bereiches elastisch ist. Vorzugsweise sind diese elastischen Mittel als peripher an einem Dorn angeordnetes Federblatt ausgebildet, wobei der Federweg zwischen 0,1 mm und 4,0 mm beträgt.

In wiederum einer anderen Ausführungsform sind die zwei Teile plattenförmig ausgestaltet, so dass der Platzbedarf des interspinalen Implantates möglichst gering ist.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Ansicht einer Ausführungsform des erfindungsgemässen Implantates mit distrahierten Dornen;
Fig. 2 einen perspektivischen Schnitt durch eine Ausführungsform des erfindungsgemässen Implantates mit distrahierten Dornen;
Fig. 3 einen zur Zentralachse orthogonalen Querschnitt des zweiten Domes der in Fig. 2 dargestellten Ausführungsform des erfindungsgemässen Implantates; und
Fig. 4 eine perspektivische Ansicht einer Ausführungsform des erfindungsgemässen Implantates zwischen den Dornfortsätzen zweier benachbarter Wirbelkörper.

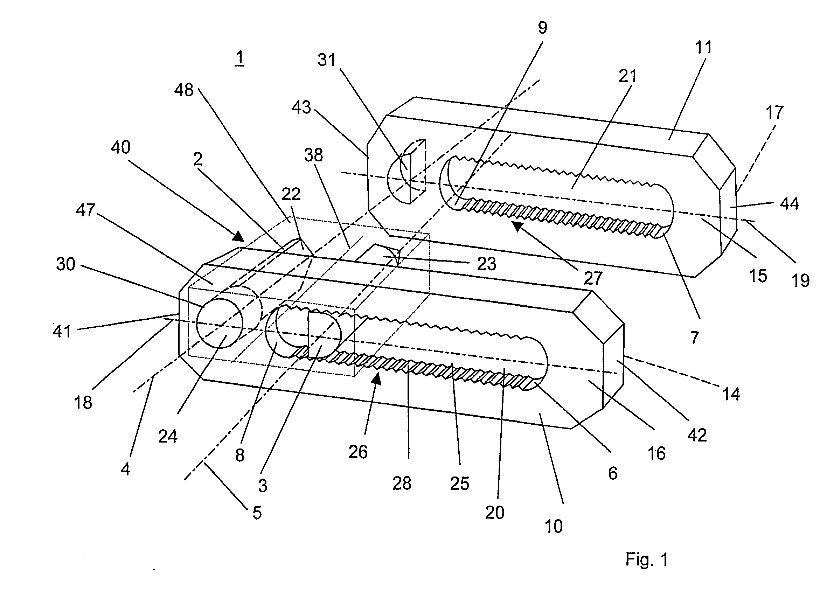

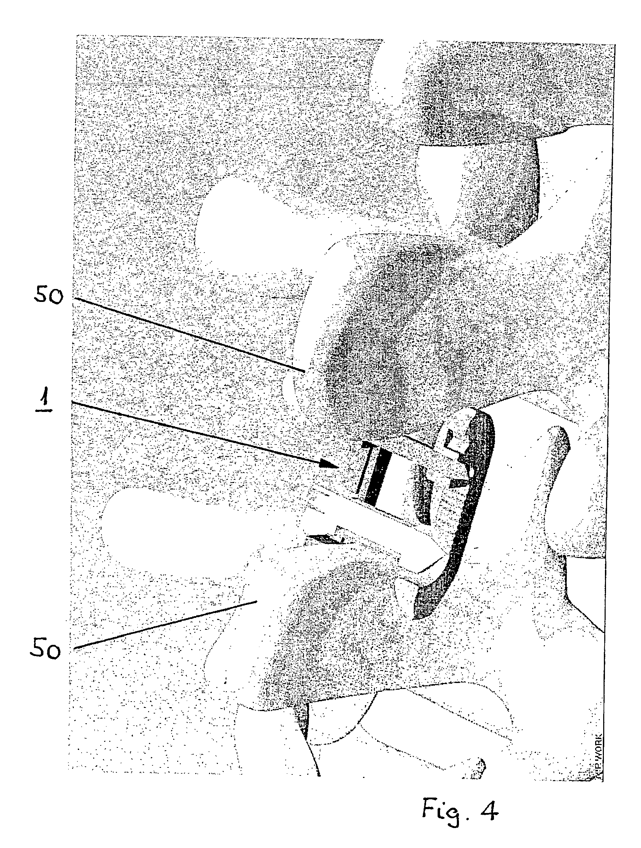

Die in Fig. 1 und 4 gezeigte Ausführungsform des interspinalen Implantates 1 umfasst im wesentlichen zwei als Platten ausgebildete Teile 10;11 mit je einer Längsachse 18;19 und je zwei die Längsachsen 18;19 schneidenden Enden 41;42;43;44. Die zwei Teile 10;11 sind parallel zueinander angeordnet und durch ein Mittelteil 40 relativ zueinander fixierbar. Das Mittelteil 40 ist derart zwischen den zwei Teilen 10;11 angeordnet, dass seine Zentralachse 38 quer zu den Längsachsen 18;19 der zwei Teile 10;11 verläuft und je ein Teil 10;11 an je einem Ende 47;48 des Mittelteiles 40 befestigbar ist. Die zwei Teile 10;11 weisen je eine durch die je zwei Enden 41 ;42;43;44 seitlich begrenzten Appositionsfläche 14;15 auf, welche auf je einer Seite der Dornfortsätze 50 (Fig. 4) zur Anlage bringbar sind. Jedes Teil 10;11 weist ein Langloch 20;21 mit einer zu der Längsachse 18;19 des entsprechenden Teiles 10;11 parallelen langen Achse auf, wobei jedes der zwei Teile 10;11 vom entsprechenden Langloch 20;21 von der Appositionsfläche 14;15 bis zur gegenüberliegenden, aussenstehenden Oberfläche 16;17 durchdrungen wird. Das Mittelteil 40 ist parallel zu den Längsachsen 18;19 der zwei Teile 10;11 distrahierbar und durch zwei Dorne 2;3 ausgestaltet, deren Längsachsen 4;5 quer zu den Appositionsflächen 14;15 stehen. Jedes der zwei Langlöcher 20;21 weist ein die Längsachse 18;19 des entsprechenden Teiles 10;11 schneidendes, erstes Ende 6;7 und ein ebenfalls die Längsachsen 18;19 des entsprechenden Teiles 10;11 schneidendes, zweites Ende 8;9 auf. Ferner weist jedes der zwei Teile 10;11 eine axial von den Langlöchern 20;21 abgegrenzte Öffnung 30;31 auf, wovon je eine Öffnung 30;31 je eines der zwei Teile 10;11 von der Appositionsfläche 14;15 zur aussenstehenden Oberfläche 16;17 durchdringt. Die zwei Öffnungen 30;31 sind koaxial zur Längsachse 4 des ersten Domes 2 angeordnet, so dass der erste Dorn 2 mit seinem hinteren Ende 24 in die erste Öffnung 30 einführbar mit dem ersten Teil 10 fest verbindbar ist und während der Implantation durch die zweite Öffnung 31 im zweiten Teil 11 durchführbar und dort fixierbar ist. Der zweite Dorn 3 ist bei den zweiten Enden 8;9 der Langlöcher 20;21 durch diese durchgeführt und mittels eines Distraktionsinstrumentes (nicht gezeichnet) parallel zu den Längsachsen 18;19 der zwei Teile 10;11 in den zwei Langlöchern 20;21 verschiebbar. An den parallel zu den Längsachsen 18;19 stehenden Seitenflächen 26;27 der Langlöcher 20;21 sind Verzahnungen 28 angebracht, so dass der zweite Dorn 3 in den Langlöchern 20;21 durch axiale Stauchung formschlüssig fixierbar ist.

Die Dorne 2;3 sind derart ausgestaltet, dass sie eine zur ihrer Längsachse 4;5 orthogonale, kreissegmentförmige Querschnittsfläche aufweisen, wobei die ebenen Seitenflächen der zwei Dorne 2;3 gegeneinander gerichtet sind und senkrecht zu den Längsachsen 18;19 der zwei Teile 10;11 stehen. Ferner sind die zwei Dorne 2;3 an ihren vorderen Enden 22;23 spitzig ausgebildet.

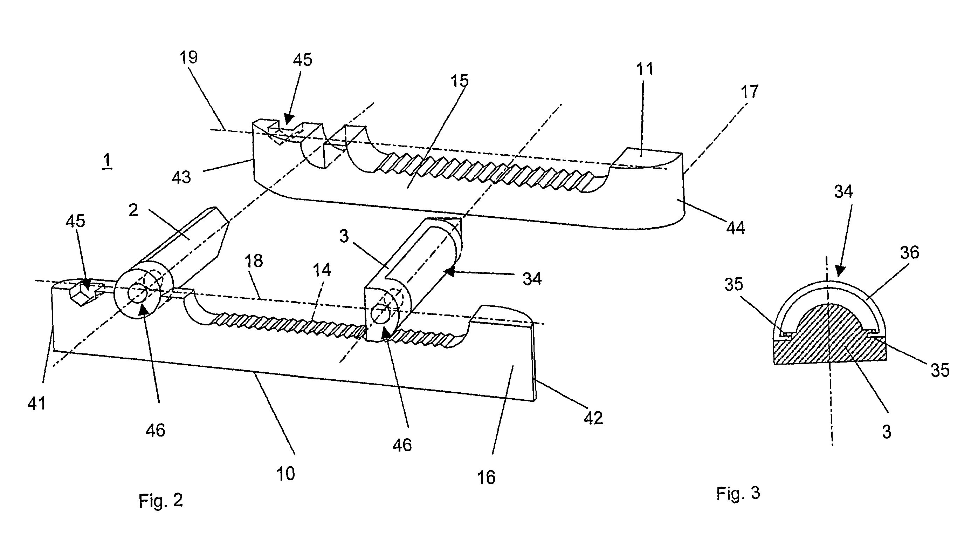

Fig. 2 zeigt eine Ausführungsform der interspinalen Prothese 1, welche sich von der in Fig. 1 dargestellten Ausführungsform nur darin unterscheidet, dass der zweite Dorn 3 mit elastischen Mitteln 34 ausgestattet ist. Ferner sind die Appositionsflächen 14;15 an den die Längsachsen 18;19 der zwei Teile 10;11 schneidenden, seitlichen Enden 41;42;43;44 der zwei Teile 10;11 gekrümmt, wodurch eine bessere Anpassung der Appositonsflächen 14;15 an die Anatomie erreichbar ist. Bei den ersten Enden 41;43 der zwei Teile 10;11 sind von den aussenstehenden Oberflächen 16;17 her eindringende, erste Mittel 45 zur Aufnahme der Spitzen eines Instrumente (nicht gezeichnet) angebracht. Diese ersten Mittel 45 zur Aufnahme der Instrumentenspitzen sind hier als Innensechskante ausgebildet, so dass jedes der Teile 10;11 zur Implantation an den Instrumentenspitzen anbringbar ist, ohne dass es sich relativ zu den Instrumentenspitzen verschieben oder rotieren kann. An den zwei Dornen 2;3 sind zweite Mittel 46 zur Aufnahme der Spitzen eines Instrumentes (nicht gezeichnet) angebracht, welche hier als von den hinteren Enden 24;25 der zwei Dorne 2;3 eindringende Bohrungen ausgebildet sind. In diese zweiten Mittel 46 zur Aufnahme von Instrumentenspitzen sind die Spitzen eines Distraktionsinstrumentes einführbar, mittels welchem der zweite Dorn 3 parallel zu den Längsachsen 18;19 der zwei Teile 10;11 relativ zum ersten Dorn 2 verschiebbar ist.

Der zweite Dorn 3 umfasst an seiner an den Dornfortsatz eines Wirbelkörpers angrenzenden Oberfläche elastische Mittel 34, welche nach der Implantation eine Bewegung der an den Dornen 2;3 anstehenden Dornfortsätze gegeneinander ermöglichen. Wie in Fig. 3 gezeigt, bestehen die elastischen Mittel 34 im wesentlichen aus einem bogenförmigen Federblatt 36, welches zwischen den zwei Appositionsflächen 14;15 in zwei radialen Nuten 35 im zweiten Dorn 3 eingespannt ist und radial elastisch deformierbar ist. Ferner ist die zur Längsachse 5 des zweiten Dornes 3 orthogonale Querschnittsfläche des Dornes 3 im Bereich des Federblattes 36 verkleinert, so dass eine Einfederung des Federblattes 36 ermöglicht wird.

Beschreibung des Implantationsvorganges:
Nachdem die Inzission ausgeführt wurde und nur die Muskulatur an den Seiten der Processi Spinosi abgelöst worden sind, kann das interspinale Implantat 1 implantiert werden. Für die Implantation erübrigt sich aufgrund der an den vorderen Enden 22;23 spitzigen Ausgestaltung der Dorne 2;3 eine Entfernung der Ligamente im Bereich der zu behandelnden Wirbelkörper. Die zwei Dorne 2;3 werden zur Implantation mit ihren hinteren Enden 24;25 in das erste Teil 10 eingeführt und parallel zur Längsachse 18 des ersten Teiles 10 soweit gegeneinander verschoben, bis sie ein Mittelteil 40 mit möglichst geringem Querschnitt bilden. Das erste Teil 10 wird zusammen mit den zwei Dornen 2;3 mit der einen Klemmbacke eines Instrumentes verbunden, während das zweite Teil 11 mit der zweiten Klemmbacke desselben Instrumentes verbunden wird. Das interspinale Implantat 1 wird so eingeführt, dass die mit dem ersten Teil 10 verbunden Dorne 2;3 links von den Processi Spinosi und den Ligamenti interspinale positioniert sind und das zweite Teil 11 rechts von den Processi Spinosi positioniert ist. Das interspinale Implantat 1 wird möglichst weit ventral positioniert. Mittels des Instrumentes werden die Dorne 2;3 durch die Ligamente gestossen und mit den vorderen Enden 22;23 durch das Langloch 21, respektive die zweite Öffnung 31 im zweiten Teil 11 durchgeführt. Anschliessend werden mittels eines zweiten Instrumentes die Dorne 2;3 parallel zu den Längsachsen 18;19 der zwei Teile 10;11 distrahiert. Da die Dorne 2;3 zwischen zwei benachbarten Processi Spinose angeordnet sind und während der Distraktion an diesen zur Anlage kommen, werden durch weiteres Distrahieren der zwei Dorne 2;3 die zwei angrenzenden Wirbelkörper distrahiert. Nachdem die gewünschte Distraktion der zwei angrenzenden Wirbelkörper erreicht wurde, werden die zwei Dorne 2;3 durch zusammenpressen der Klemmbacken des Instrumentes in den Langlöchern 20;21 respektive den zwei Öffnungen 30;31 verquetscht, so dass das vordere, respektive hintere Ende 23;25 des zweiten Domes 3 in die Verzahnungen 28 eingepresst werden und die Dorne 2;3 formschlüssig mit den zwei Teilen 10;11 verbunden sind. Nachdem die Fixierung der Dorne 2;3 in den zwei Teilen 10;11 erfolgt ist, kann das Instrument entfernt und die Wunde geschlossen werden.

## Patentansprüche

1. Interspinales Implantat (1) mit
A) einem in den Interspinalraum einführbaren Mittelteil (40) mit einer Zentralachse (38), einem ersten, die Zentralachse (38) schneidenden Ende (47) und einem zweiten, die Zentralachse (38) schneidenden Ende (48); und
B) zwei longitudinalen Teilen (10;11) mit je einer quer zur Zentralachse (38) des Mittelteiles (37) stehenden Längsachse (18;19) und je einer quer zur Zentralachse (38) des Mittelteiles (37) gerichteten Appositionsfläche (14;15) zur Anlage an zwei Dornfortsätze zweier benachbarter Wirbelkörper, wobei
C) je ein Teil (10;11) mit je einem Ende (14;15) des Mittelteiles (40) derart verbindbar ist, dass die Appositionsflächen (14;15) gegeneinander gerichtet sind, und
D) das Mittelteil (40) quer zu seiner Zentralachse (38) entlang der Längsachsen (18; 19) der zwei Teile (10;11) distrahierbar ist,
**dadurch gekennzeichnet, dass**
E) das Mittelteil (40) zwei relativ zueinander verschiebbare Dorne (2;3) mit je einer zur Zentralachse (38) des Mittelteiles (40) parallelen Längsachse (4;5) umfasst, wobei
F) die Dorne (2,3) je ein vorderes und je ein hinteres Ende (22;23;24;25) aufweisen und an ihren vorderen Enden (22,23) spitzig ausgestaltet sind.

2. Interspinales Implantat (1) nach Anspruch 1, **dadurch gekennzeichnet, dass** die zwei Dorne (2;3) mit ihren hinteren Enden (24;25) am ersten Teil (10) und mit ihren vorderen Enden (22;23) am zweiten Teil (11) in einer gewünschten Position fixierbar sind.

3. Interspinales Implantat (1) nach Anspruch 2, **dadurch gekennzeichnet, dass** die zwei Dorne (2;3) mit ihren Enden (22;23;24;25) formschlüssig am ersten und zweiten Teil (10;11) fixierbar sind.

4. Interspinales Implantat (1) nach einem der Ansprüche 1 bis 3, **dadurch gekennzeichnet, dass** jedes der zwei Teile (10;11) seiner Appositionsfläche (14;15) gegenüberliegend eine aussenstehende Oberfläche (16;17) aufweist und je ein von seiner Appositionsfläche (14;15) zu seiner aussenstehenden Oberfläche (16;17) durchgehendes Langloch (20;21) umfasst, dessen lange Achsen parallel zu den Längsachsen (18;19) der zwei Teile (10;11) sind.

5. Interspinales Implantat (1) nach Anspruch 4, **dadurch gekennzeichnet, dass** in den Langlöchern (20;21) mindestens ein Dorn (2;3) parallel zu den Längsachsen (18;19) der zwei Teile (10;11) verschiebbar ist.

6. Interspinales Implantat (1) nach einem der Ansprüche 1 bis 5, **dadurch gekennzeichnet, dass** jeder der Dorne (2;3) eine maximale diametrale Abmessung zwischen 2 mm und 5 mm aufweist.

7. Interspinales Implantat (1) nach einem der Ansprüche 1 bis 6, **dadurch gekennzeichnet, dass** der zweite Dorn (3) parallel zu den Längsachsen (18;19) der zwei Teile (10;11) zwischen 7 mm und 15 mm relativ zum ersten Dorn (2) verschiebbar ist.

8. Interspinales Implantat (1) nach einem der Ansprüche 4 bis 7, **dadurch gekennzeichnet, dass** jedes Langloch (20;21) zu den Längsachsen (18;19) der zwei Teile (10;11) parallele Seitenflächen (26;27) mit einer dreidimensionalen, makroskopischen Struktur aufweist.

9. Interspinales Implantat (1) nach Anspruch 8, **dadurch gekennzeichnet, dass** die dreidimensionale, makroskopische Struktur durch eine Verzahnung (28) ausgestaltet ist.

10. Interspinales Implantat (1) nach einem der Ansprüche 1 bis 9, **dadurch gekennzeichnet, dass** die zwei Teile (10;11) aus einem härteren Material als die zwei Dorne (2;3) hergestellt sind.

11. Interspinales Implantat (1) nach einem der Ansprüche 1 bis 10, **dadurch gekennzeichnet, dass** der erste Dorn (2) an seinem hinteren Ende (24) fest mit einem der zwei Teile (10;11) verbunden ist.

12. Interspinales Implantat (1) nach einem der Ansprüche 1 bis 11, **dadurch gekennzeichnet, dass** mindestens einer der zwei Dorne (2;3) elastische Mittel (34) umfasst, mittels welcher der Dorn (2;3) quer zu seiner Längsachse (4;5) elastisch deformierbar ist.

13. Interspinales Implantat (1) nach Anspruch 12, **dadurch gekennzeichnet, dass** die elastischen Mittel (34) durch ein peripher an dem mindestens einen Dorn (2;3) angeordneten Federblatt (36) ausgestaltet sind.

14. Interspinales Implantat (1) nach Anspruch 12 oder 13, **dadurch gekennzeichnet, dass** die elastischen Mittel (34) parallel zu den Längsachsen (18;19) der zwei Teile (10;11) zwischen 0,1 mm und 4,0 mm elastisch deformierbar sind.

15. Interspinales Implantat (1) nach einem der Ansprüche 1 bis 14, **dadurch gekennzeichnet, dass** die zwei Teile (10;11) plattenförmig ausgestaltet sind.

## Claims

1. Interspinal implant (1) with
A) a middle part (40), which can be introduced into the interspinal space, with a central axis (38), a first end (47) intersecting the central axis (38) and a second end (48) intersecting the central axis (38), and.
B) two longitudinal parts (10; 11), each with a longitudinal axis (18; 19), extending transversely to the central axis (38) of the middle part (37), and each with an apposition surface (14; 15), directed transversely to the central axis (38) of the middle part (37), for contacting two processi spinosi of two adjacent bodies of vertebrae,
C) each part (10; 11) being connectable with an end (14; 15) of the middle part (40) in such a manner, that the apposition surfaces (14; 15) are directed against one another; and
D) the middle part (40) can be extended transversely to its central axis (38) along the longitudinal axes (18; 19) of the two parts (10; 11),
**characterized in that**
the middle part (40) comprises two pins (2; 3), which can be shifted relative to one another, each with a longitudinal axis (4; 5) parallel to the central axis (38) of the middle part (40), wherein
F) the pins (2; 3) each have a front and a rear end (22; 23; 24; 25) and are pointed at their front ends (22; 23).

2. The interspinal implant (1) of claim 1, **characterized in that** the two pins (2; 3) can be immobilized in a desired position with their rear ends (24; 25) at the first part (10) and with their front ends (22; 23) at the second part (11).

3. The interspinal implant (1) of claim 2, **characterized in that** the two pins (2; 3) can be immobilized with their rear ends (22; 23; 24; 25) positively at the two parts (10; 11).

4. The interspinal implant (1) of one of the claims 1 to 3, **characterized in that** each of the two parts (10; 11) has an external surface (16; 17) opposite to its apposition surface (14; 15) and each comprises an elongated hole (20; 21), which extends from its apposition surface (14; 15) to its external surface (16; 17) and the long axes of which are parallel to the longitudinal axes (18; 19) of the two parts (10; 11).

5. The interspinal implant (1) of claim 4, **characterized in that** at least one pin (2; 3) can be shifted parallel to the longitudinal axes (18; 19) of the two parts (10; 11) in the elongated holes (20; 21).

6. The interspinal implant (1) of one of the claims 1 to 5, **characterized in that** each of the pins (2; 3) has a maximum diametric dimension of between 2 mm and 5 mm.

7. The interspinal implant (1) of one of the claims 1 to 6, **characterized in that** the second pin (3) can be shifted a distance of between 7 mm and 15 mm relative to the first pin (2) parallel to the longitudinal axes (18; 19) of the two parts (10; 11).

8. The interspinal implant (1) of one of the claims 4 to 7, **characterized in that** each elongated hole (20; 21) has side surfaces (26; 27), which are parallel to the longitudinal axes (18; 19) of the two parts (10; 11) and have been provided with a three-dimensional, macroscopic structure.

9. The interspinal implant (1) of claim 8, **characterized in that** the three-dimensional, macroscopic structure is configured as teeth (28).

10. The interspinal implant (1) of one of the claims 1 to 9, **characterized in that** the two parts (10; 11) are produced from a material, which is harder than the material of the two pins (2; 3).

11. The interspinal implant (1) of one of the claims 1 to 10, **characterized in that** the first pin (2) is connected at its rear end (24) firmly with one of the two parts (10; 11).

12. The interspinal implant (1) of one of the claims 1 to 11, **characterized in that** at least one of the two pins (2; 3) comprises elastic means (34), by means of which the pin (2; 3) can be deformed elastically transversely to its longitudinal axis (4; 5).

13. The interspinal implant (1) of claim 12, **characterized in that** the elastic means (34) are formed by a leaf spring (36) disposed peripherally at the at least one pin (2; 3).

14. The interspinal implant (1) of claims 12 or 13, **characterized in that** the elastic means (34) can be deformed elastically by between 0.1 mm and 4.0 mm parallel to the longitudinal axes (18; 19) of the two parts (10; 11).

15. The interspinal implant (1) of one of the claims 1 to 14, **characterized in that** the two parts (10; 11) are configured in the shape of a panel.

## Revendications

1. Implant intervertébral (1) comprenant :
A) un élément central ayant un axe central (38) pouvant être inséré dans l'espace intervertébral (40), dont une première extrémité (47) coupe l'axe central (38) et une deuxième extrémité (48) coupe l'axe central (38) ; et
B) deux éléments longitudinaux (10 ; 11) présentant chacun un axe longitudinal (18 ; 19) transversalement à l'axe central (38) de l'élément central (37) et des surfaces d'apposition (14 ; 15) transversalement à l'axe central (38) de l'élément central (37) pouvant être mises en appui sur deux apophyses épineuses de deux corps vertébraux adjacents, dans lequel
C) chaque élément (10 ; 11) peut être relié respectivement à une extrémité (14 ; 15) de l'élément central (40) de telle sorte que les surfaces d'apposition (14 ; 15) se situent en face l'une de l'autre, et
D) l'élément central (40) peut être étirée transversalement à l'axe central (38) le long des axes longitudinaux (18 ; 19) des deux éléments (10 ; 11),
**caractérisé en ce que**
E) l'élément central (40) comprend deux tourillons (2 ; 3) déplaçables l'un par rapport à l'autre, chacun présentant un axe longitudinal (4 ; 5) parallèle à l'axe central (38) de l'élément central (40) dans lequel
F) les tourillons (2,3) présentent chacun une extrémité avant et une extrémité arrière (22 ; 23 ; 24 ; 25) et les extrémités avant (22 ; 23) sont réalisées en forme de pointe.

2. Implant intervertébral (1) selon la revendication 1, **caractérisé en ce que** les deux tourillons (2 ; 3) peuvent être fixés en toute position souhaitée au premier élément (10) par l'intermédiaire de leurs extrémités arrières (24 ; 25) et au deuxième élément (11) par l'intermédiaire de leurs extrémités avant (22 ; 23).

3. Implant intervertébral (1) selon la revendication 2, **caractérisé en ce que** les deux tourillons (2 ; 3) peuvent être fixés par emboîtement au premier et deuxième élément (10 ; 11) par l'intermédiaire de leurs extrémités (22 ; 23 ; 24 ; 25).

4. Implant intervertébral (1) selon l'une quelconque des revendications 1 à 3, **caractérisé en ce que** chacun des deux (10 ; 11) présente une surface externe (16 ; 17) opposée à sa surface d'apposition (14 ; 15) et chacune comprend un trou oblong (20 ; 21) s'étendant de sa surface d'apposition (14 ; 15) jusqu'à sa surface externe (16 ; 17), dont les longs axes sont parallèles aux axes longitudinaux (18 ; 19) des deux éléments (10 ; 11).

5. Implant intervertébral (1) selon la revendication 4, **caractérisé en ce qu'**au moins un tourillon (2 ;3) peut être déplacé dans les trous oblongs (20 ; 21) parallèlement aux axes longitudinaux (18 ; 19) des deux éléments (10 ; 11).

6. Implant intervertébral (1) selon l'une quelconque des revendications 1 à 5, **caractérisé en ce que** chaque tourillon (2 ; 3) présente un diamètre maximal compris entre 2 mm et 5 mm.

7. Implant intervertébral (1) selon l'une quelconque des revendications 1 à 6, **caractérisé en ce que** le deuxième tourillon (3) peut être déplacé parallèlement aux axes longitudinaux (18 ; 19) des deux éléments (10 ; 11) sur une distance comprise entre 7 mm et 15 mm par rapport au premier tourillon (2).

8. Implant intervertébral (1) selon l'une quelconque des revendications 4 à 7, **caractérisé en ce que** chaque trou oblong (20 ; 21) présente des faces latérales (26 ; 27) ayant une structure macroscopique tridimensionnelle, parallèles aux axes longitudinaux (18 ; 19) des deux éléments (10 ; 11).

9. Implant intervertébral (1) selon la revendication 8, **caractérisé en ce que** la structure macroscopique tridimensionnelle est pourvue d'une denture (28).

10. Implant intervertébral (1) selon l'une quelconque des revendications 1 à 9, **caractérisé en ce que** les deux éléments (10 ; 11) sont fabriqués à partir d'un matériau plus dur que les deux tourillons (2 ; 3).

11. Implant intervertébral (1) selon l'une quelconque des revendications 1 à 10, **caractérisé en ce qu'**au niveau de son extrémité arrière (24), le premier tourillon (2) est fermement fixé à l'un des deux éléments (10 ; 11).

12. Implant intervertébral (1) selon l'une quelconque des revendications 1 à 11, **caractérisé en ce qu'**au moins un des deux tourillons (2 ; 3) comprend des moyens élastiques (34) permettant une déformation élastique transversalement à l'axe longitudinal (4 ; 5) du tourillon (2 ; 3).

13. Implant intervertébral (1) selon la revendication 12, **caractérisé en ce que,** les moyens élastiques (34) sont formés par une lame de ressort (36) placée à la périphérie d'au moins un tourillon (2 ; 3).

14. Implant intervertébral (1) selon la revendication 12 ou 13, **caractérisé en ce que** les moyens élastiques (34) sont élastiquement déformables selon 0,1 mm à 4,0 mm parallèlement aux axes longitudinaux (18 ; 19) des deux éléments (10 ; 11).

15. Implant intervertébral (1) selon l'une quelconque des revendications 1 à 14, **caractérisé en ce que** les deux éléments (10 ; 11) sont réalisés en forme de plaque.
